# EUROPEAN PATENT APPLICATION

(11) **EP 3 845 558 A1**
(43) Date of publication of application: **07.07.2021**
(21) Application number: 19855038.6
(22) Date of filing: 29.08.2019
(51) Int. Cl.: C07K 16/00, C12N 15/13

(54) **ANTIBODY HALF-MOLECULE, AND METHOD FOR INHIBITING HOMODIMER FORMATION OF ANTIBODY HALF-MOLECULE**

(30) Priority: 29.08.2018 JP 2018160733
(71) Applicant: CHUGAI SEIYAKU KABUSHIKI KAISHA, Tokyo 115-8543 (JP)
(72) Inventor: KAWA, Tatsuya, Gotemba-shi, Shizuoka 412-8513 (JP)
(74) Representative: Vossius & Partner Patentanwälte Rechtsanwälte mbB
(86) International application number: PCT/JP2019/033830
(87) International publication number: WO 2020/045545

(57) **Abstract**

The present invention relates to antibody half molecules, and methods for inhibiting homodimerization of antibody half molecules.

SEQUENCE LISTING

<110>  CHUGAI SEIYAKU KABUSHIKI KAISHA

<120>  ANTIBODY HALF-MOLECULE, AND METHOD FOR INHIBITING
       HOMODIMER FORMATION OF ANTIBODY HALF-MOLECULE

<130>  AE1258 EP S3

<140>  EP 19 85 5038.6
<141>  2019-08-29

<150>  JP 2018-160733
<151>  2018-08-29

<160>  32

<170>  PatentIn version 3.5

<210>  1
<211>  119
<212>  PRT
<213>  Artificial Sequence

<220>
<223>  MRAH

<400>  1

<210>  2
<211>  328

<212>  PRT
<213>  Artificial sequence

<220>
<223>  G1d

<400>  2

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  3
<211>  107
<212>  PRT
<213>  Artificial Sequence

<220>
<223>  MRAL

<400>  3

<210> 4
<211> 107
<212> PRT
<213> Artificial Sequence

<220>
<223> k0

<400> 4

<210> 5
<211> 116
<212> PRT
<213> Artificial Sequence

<220>
<223> EGLVH

<400> 5

Gln Asp Gln Leu Val Gln Ser Gly Ala Glu Val Lys Lys Pro Gly Ala
1               5                   10                  15

Ser Val Lys Val Ser Cys Lys Ala Ser Gly Phe Asn Ile Lys Asp Thr
            20                  25                  30

Tyr Ile Gln Trp Val Arg Gln Ala Pro Gly Gln Gly Leu Glu Trp Met
        35                  40                  45

Gly Arg Ile Asp Pro Leu Arg Lys Gln Thr Lys Tyr Arg Glu Lys Phe
    50                  55                  60

Glu Gly Arg Val Thr Ile Thr Ala Asp Thr Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Val Arg Ser Gly Arg Glu Phe Asp Tyr Trp Gly Gln Gly Thr Leu Val
            100                 105                 110

Thr Val Ser Ser
        115

<210>  6
<211>  327
<212>  PRT
<213>  Artificial Sequence

<220>
<223>  wtIgG4C4

<400>  6

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Tyr Ile Thr Arg Glu Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Phe Pro Pro Ser Gln Glu Glu Met Thr Lys
225                 230                 235                 240

Asn Gln Val Ser Leu Arg Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Lys Pro Val Leu Asp Ser Asp Gly Ser Phe Arg Leu Glu Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  7
<211>  106
<212>  PRT
<213>  Artificial Sequence

<220>
<223>  EGLVL

<400>  7

Asp Ile Gln Met Thr Gln Ser Pro Ser Ser Leu Ser Ala Ser Val Gly

1               5                   10                  15

Asp Arg Val Thr Ile Thr Cys Arg Ala Ser Gln Asp Ile His Lys Tyr
            20                  25                  30

Ile Ala Trp Tyr Gln Gln Lys Pro Gly Gln Ala Pro Arg Leu Leu Ile
        35                  40                  45

Gln Tyr Thr Ser Thr Leu Gln Pro Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Tyr Cys Leu Gln Tyr Glu Gln Leu Arg Thr
                85                  90                  95

Phe Gly Gly Gly Thr Lys Val Glu Ile Lys
            100                 105

<210>  8
<211>  214
<212>  PRT
<213>  Artificial Sequence

<220>
<223>  MRAL-k0

<400>  8

<210> 9
<211> 447
<212> PRT
<213> Artificial Sequence

<220>
<223> MRAH-G1d.dh3.dCH3

<400> 9

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210> 10
<211> 447
<212> PRT
<213> Artificial Sequence

<220>
<223> MRAH-G1d.dh3.dCH3.K334A

<400> 10

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Ala
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  11
<211>  447
<212>  PRT
<213>  Artificial Sequence

<220>
<223>  MRAH-G1d.dh3.dCH3.K334C

<400>  11

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Cys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  12
<211>  447
<212>  PRT
<213>  Artificial Sequence

<220>
<223>  MRAH-G1d.dh3.dCH3.K334D

<400>  12

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Asp
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  13
<211>  447
<212>  PRT
<213>  Artificial Sequence

<220>
<223>  MRAH-G1d.dh3.dCH3.K334E

<400>  13

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Glu
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210> 14
<211> 447
<212> PRT
<213> Artificial Sequence

<220>
<223> MRAH-G1d.dh3.dCH3.K334F

<400> 14

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
        180                 185                 190

Ser Ser Leu Gly Thr Gln Thr Tyr Ile Cys Asn Val Asn His Lys Pro
    195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Phe
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  15
<211>  447
<212>  PRT
<213>  Artificial Sequence

<220>
<223>  MRAH-G1d.dh3.dCH3.K334G

<400>  15

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Gly
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  16
<211>  447
<212>  PRT
<213>  Artificial Sequence

<220>
<223>  MRAH-G1d.dh3.dCH3.K334H

<400>  16

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu His
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  17
<211>  447

<212>  PRT
<213>  Artificial Sequence

<220>
<223>  MRAH-G1d.dh3.dCH3.K334I

<400>  17

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Ile
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  18
<211>  447
<212>  PRT
<213>  Artificial Sequence

<220>

<223>  MRAH-G1d.dh3.dCH3.K334L

<400>  18

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Leu
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  19
<211>  447
<212>  PRT
<213>  Artificial Sequence

<220>
<223>  MRAH-G1d.dh3.dCH3.K334M

<400>  19

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Met
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  20
<211>  447
<212>  PRT
<213>  Artificial Sequence

<220>
<223>  MRAH-G1d.dh3.dCH3.K334N

<400>  20

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30
His Ala Trp Ser Trp Val Arg Gln Pro Pro Gly Arg Gly Leu Glu Trp
        35                  40                  45
Ile Gly Tyr Ile Ser Tyr Ser Gly Ile Thr Thr Tyr Asn Pro Ser Leu
    50                  55                  60
Lys Ser Arg Val Thr Met Leu Arg Asp Thr Ser Lys Asn Gln Phe Ser
65                  70                  75                  80
Leu Arg Leu Ser Ser Val Thr Ala Ala Asp Thr Ala Val Tyr Tyr Cys
                85                  90                  95
Ala Arg Ser Leu Ala Arg Thr Thr Ala Met Asp Tyr Trp Gly Gln Gly
            100                 105                 110
Ser Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Ser Pro Pro Ser Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Asn
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  21
<211>  447
<212>  PRT
<213>  Artificial Sequence

<220>
<223>  MRAH-G1d.dh3.dCH3.K334P

<400>  21

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Pro
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  22
<211>  447
<212>  PRT
<213>  Artificial Sequence

<220>
<223>  MRAH-G1d.dh3.dCH3.K334Q

<400>  22

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Gln
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  23
<211>  447
<212>  PRT
<213>  Artificial Sequence

<220>
<223>  MRAH-G1d.dh3.dCH3.K334R

<400>  23

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Arg
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  24
<211>  447
<212>  PRT
<213>  Artificial Sequence

<220>
<223>  MRAH-G1d.dh3.dCH3.K334S

<400>  24

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Ser
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  25
<211>  447
<212>  PRT
<213>  Artificial Sequence

<220>
<223>  MRAH-G1d.dh3.dCH3.K334T

<400>  25

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Thr
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210> 26
<211> 447
<212> PRT
<213> Artificial Sequence

<220>
<223> MRAH-G1d.dh3.dCH3.K334V

<400> 26

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Val
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  27
<211>  447
<212>  PRT
<213>  Artificial Sequence

<220>
<223>  MRAH-G1d.dh3.dCH3.K334W

<400>  27

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Trp
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  28
<211>  447
<212>  PRT
<213>  Artificial Sequence

<220>
<223>  MRAH-G1d.dh3.dCH3.K334Y

<400>  28

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Tyr
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Tyr Leu
385                 390                 395                 400

Asp Ser Asp Gly Ser Phe Phe Leu Tyr Ser Asp Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  29
<211>  330
<212>  PRT
<213>  Homo sapiens

<400>  29

<210>  30
<211>  326
<212>  PRT
<213>  Homo sapiens

<400>  30

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  31
<211>  377
<212>  PRT
<213>  Homo sapiens

<400>  31

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375


<210>  32
<211>  327
<212>  PRT
<213>  Homo sapiens

<400>  32

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser

290                 295                 300

Cys Ser Val Met His Glu Ala Leu His Asn His Tyr Thr Gln Lys Ser
305                 310                 315                 320

Leu Ser Leu Ser Leu Gly Lys
                325

## Description

### [Technical Field]

The present invention relates to antibody half molecules, and methods for inhibiting homodimerization of antibody half molecules.

### [Background Art]

Patent Document 1 describes that a monomer polypeptide containing a mutated Fc region is used for diagnostic or therapeutic purposes, wherein the mutated Fc region has one or more amino acid substitutions within or in proximity to the CH3 region interface in the Fc region, wherein the amino acid substitutions inhibit dimerization of the Fc region.

### [Citation List]

### [Patent Literature]

[PTL 1] WO 2012/020096

### [Summary of Invention]

### [Technical Problem]

An objective of the present invention is to provide alterations and methods by which antibody half molecules that are less likely to form homodimers can be obtained more easily.

### [Solution to Problem]

The present inventors discovered that homodimerization of two antibody half molecules can be inhibited by making alterations that prevent the two antibody half molecules from forming disulfide bonds between the hinge regions, and alterations that destabilize the interface between the CH3 regions of the two antibody half molecules so that their interaction is weakened, and further altering the amino acid residue at position 334 according to the EU numbering system in the CH2 region to an amino acid residue other than an arginine residue and a lysine residue. The present invention has thus been completed. In one embodiment, the present invention is exemplified by [1] to [21] below.
[1] An antibody half molecule comprising a hinge region, a CH2 region, and a CH3 region, wherein at least one cysteine residue in the hinge region is substituted with another amino acid residue, wherein the amino acid residue at position 334 according to the EU numbering system in the CH2 region is an amino acid residue other than an arginine residue and a lysine residue, and wherein a conformational interface in the CH3 region is destabilized.
[2] The antibody half molecule of [1], wherein homodimerization of the antibody half molecule is inhibited.
[3] The antibody half molecule of [1] or [2], wherein the amino acid residue at position 334 according to the EU numbering system is selected from the group consisting of a histidine residue, a cysteine residue, a phenylalanine residue, a glycine residue, an isoleucine residue, a methionine residue, an asparagine residue, a proline residue, a glutamine residue, a serine residue, a threonine residue, a tryptophan residue, a tyrosine residue, an alanine residue, a leucine residue, a valine residue, an aspartic acid residue, and a glutamic acid residue.
[4] The antibody half molecule of any one of [1] to [3], wherein the at least one cysteine residue is either or both of position 226 and position 229 according to the EU numbering system.
[5] The antibody half molecule of any one of [1] to [4], wherein the conformational interface in the CH3 region is destabilized by substitution of either or both of amino acid residues at position 397 and position 409 according to the EU numbering system.
[6] The antibody half molecule of any one of [1] to [5], which further comprises an antigen-binding region of H chain.
[7] The antibody half molecule of any one of [1] to [6], which further comprises a CH1 region.
[8] The antibody half molecule of [7], wherein at least one selected from the group consisting of the CH1 region, the hinge region, the CH2 region, and the CH3 region is derived from human IgG.
[9] The antibody half molecule of any one of [1] to [8], which is an antibody half molecule of a single domain antibody.
[10] The antibody half molecule of any one of [1] to [8], which further comprises an antibody L chain.
[11] The antibody half molecule of [10], wherein the L chain is derived from human IgG.
[12] A method for inhibiting homodimerization of an antibody half molecule comprising a hinge region, a CH2 region, and a CH3 region in production of the antibody half molecule, wherein the method comprises:
   (a) substitution of at least one cysteine residue in the hinge region with another amino acid;
   (b) substitution of the amino acid residue at position 334 according to the EU numbering system in the CH2 region with an amino acid residue other than an arginine residue and a lysine residue; and
   (c) destabilization of a conformational interface in the CH3 region.
[13] The method of [12], wherein (b) is substitution of the amino acid residue at position 334 according to the EU numbering system with any amino acid residue selected from the group consisting of a histidine residue, a cysteine residue, a phenylalanine residue, a glycine residue, an isoleucine residue, a methionine residue, an asparagine residue, a proline residue, a glutamine residue, a serine residue, a threonine residue, a tryptophan residue, a tyrosine residue, an alanine residue, a leucine residue, a valine residue, an aspartic acid residue, and a glutamic acid residue.
[14] The method of [12] or [13], wherein the at least one cysteine residue in (a) is either or both of position 226 and position 229 according to the EU numbering system.
[15] The method of any one of [12] to [14], wherein (c) is performed by substitution of either or both of amino acid residues at position 397 and position 409 according to the EU numbering system.
[16] The method of any one of [12] to [15], wherein the antibody half molecule further comprises an antigen-binding region of H chain.
[17] The method of any one of [12] to [16], wherein the antibody half molecule further comprises a CH1 region.
[18] The method of [17], wherein at least one selected from the CH1 region, the hinge region, the CH2 region, and the CH3 region is derived from human IgG.
[19] The method of any one of [12] to [18], wherein the antibody half molecule is a single domain antibody.
[20] The method of any one of [12] to [18], wherein the antibody half molecule further comprises an antibody L chain.
[21] The method of [20], wherein the L chain is derived from human IgG.

### Effects of the Invention

In the production of an antibody half molecule, the present invention makes the antibody half molecule less likely to dimerize and easier to obtain.

### [Brief Description of Drawings]

Fig. 1 shows the result of size exclusion chromatography that confirmed whether antibody half molecules form a dimer (whole antibody). W and H indicate the positions where the whole antibody and the antibody half molecule were eluted, respectively.
Fig. 2-1 shows the result of size exclusion chromatography that confirmed whether antibody half molecules form a dimer (whole antibody). W and H indicate the positions where the whole antibody and the antibody half molecule were eluted, respectively.
Fig. 2-2 is the continuation of Fig. 2-1.

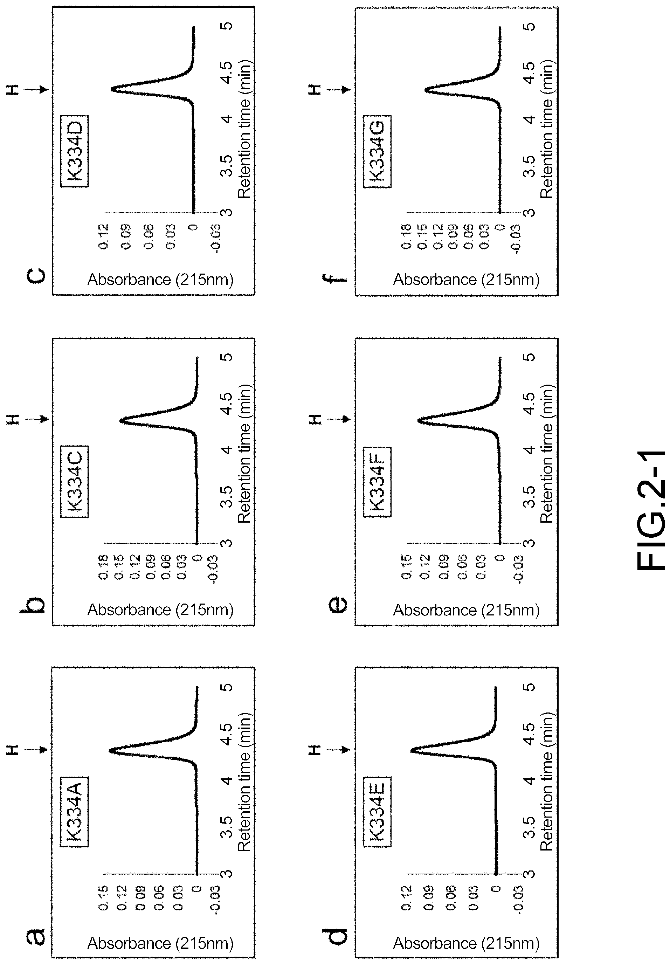

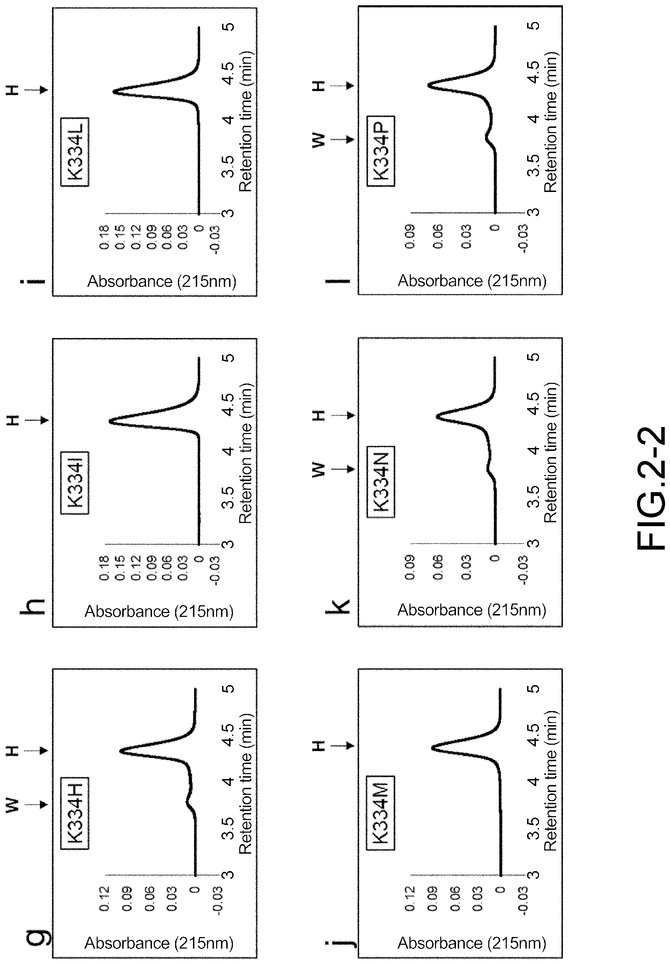

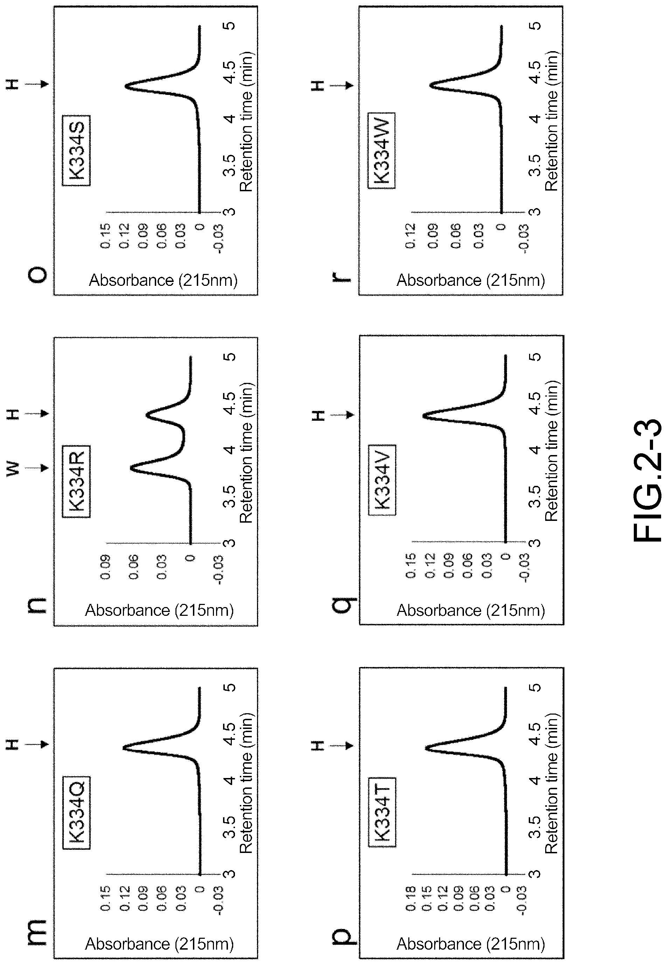

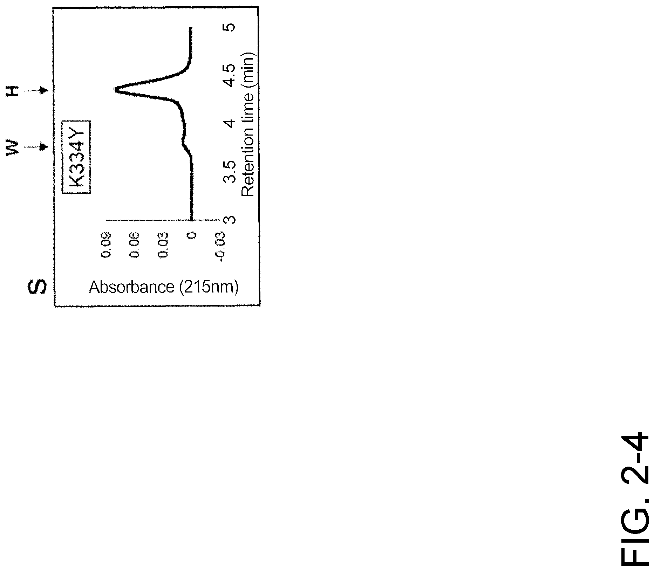

Fig. 2-3 is the continuation of Fig. 2-2.
Fig. 2-4 is the continuation of Fig. 2-3.

### [Description of Embodiments]

### 1. Definitions

Herein, the term "polypeptide" encompasses all peptides with a plurality of amino acids linked by peptide bonds. Herein, polypeptides are sometimes referred to as "peptides" or "proteins."

Herein, the term "antigen-binding region" means a compound having an activity of binding to an antigen. The antigen-binding region may be peptidic or non-peptidic.

Herein, "CH1" means a single polypeptide chain of a CH1 region of an antibody. Specifically, a CH1 region is a region represented by amino acid residues at positions 118 to 215 of an H chain according to the EU numbering system, and herein encompasses the wild-type and also variants produced by introducing amino acid residue substitutions, additions, or deletions into the wild-type.

Herein, "CH2" means a single polypeptide chain of a CH2 region of an antibody. Specifically, a CH2 region is a region represented by amino acid residues at positions 231 to 340 of an H chain according to the EU numbering system, and herein encompasses the wild-type and also variants produced by introducing amino acid residue substitutions, additions, or deletions into the wild-type.

Herein, "CH3" means a single polypeptide chain of a CH3 region of an antibody. Specifically, a CH3 region is a region represented by amino acid residues from position 341 to the C-terminus of an H chain according to the EU numbering system, and herein encompasses the wild-type and also variants produced by introducing amino acid residue substitutions, additions, or deletions into the wild-type.

Herein, "CL" means a single polypeptide chain of a CL region of an antibody. Specifically, a CL region is a region represented by amino acid residues from position 108 to the C-terminus of an L chain according to the EU numbering system, and herein encompasses the wild-type and also variants produced by introducing amino acid residue substitutions, additions or deletions into the wild-type.

A "hinge region" as used herein is a region located between CH1 and CH2 regions in an antibody. Specifically, the hinge region is a region represented by amino acid residues at positions 216 to 230 according to the EU numbering system, and herein encompasses the wild-type and also variants produced by introducing amino acid residue substitutions, additions, or deletions into the wild-type. Herein, the "hinge region portion in an antibody half molecule" means a hinge region portion in one H chain, and it means a portion consisting of a single chain polypeptide.

Herein, "antibody half molecule" means a single molecule and a fragment thereof resulting from dissociation of the binding between H chains in an antibody. Examples of an antibody half molecule in the case where the antibody is IgG include a complex composed of one H chain and one L chain. Antibody half molecules include molecules consisting of one H chain which are produced by dissociating the inter-H chain bonds of so-called heavy chain (H chain) antibodies (also called VHHs (VH originating from heavy-chain antibody)), which are antibodies consisting of two H chains and found in camelid antibodies and such (these are collectively referred to as "single domain antibodies").

In one embodiment, the antibody half molecules include those derived from chimeric antibodies or humanized antibodies.

In one embodiment, the antibody half molecules include those derived from various isotypes such as IgG, IgM, IgA, IgD, and IgE.

Herein, a "constant region" refers to a region that includes a CH1 region, a CH2 region, a CH3 region and a hinge region (H chain constant region) and/or a region that includes a CL region (L chain constant region) in an antibody. A "constant region portion in an antibody half molecule" includes either or both of the H chain constant region portion and the L chain constant region in the antibody half molecule. Examples of a constant region portion in an antibody half molecule include, but are not limited to, the H chain constant region portion of human IgG1 shown in SEQ ID NO: 29, the H chain constant region portion of human IgG2 shown in SEQ ID NO: 30, the H chain constant region portion of human IgG3 shown in SEQ ID NO: 31, and the H chain constant region portion of human IgG4 shown in SEQ ID NO: 32.

The term "Fc region" herein is used to define a C-terminal region of an immunoglobulin H chain that contains at least a portion of the constant region. The term includes native sequence Fc regions and variant Fc regions. In one embodiment, a human IgG H chain Fc region extends from Cys226, or from Pro230, to the carboxyl-terminus of the H chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins ofImmunological Interest, 5th Ed. Public Health Service, National Institutes of Health, Bethesda, MD, 1991.

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daeron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "covalent bond" herein includes all those generally known. "Covalent bonds" includes, for example, disulfide bonds and carbon-carbon bonds.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

### 2. Antibody half molecules

In one aspect, the antibody half molecules of the present invention contain a hinge region, CH2 region, and CH3 region. There is no antibody half molecule in antibodies produced from genetically unmodified B cells *in vivo* (hereinafter referred to as "native antibodies"). An antibody half molecule has at least one amino acid residue that is not conserved in the hinge region, CH2 region, or CH3 region of native antibodies.

In another aspect, the homodimerization of an antibody half molecule is inhibited. A homodimer refers to a dimer resulting from binding of two identical antibody half molecules with each other via a certain chemical bond. For example, when size exclusion chromatography is performed using a preparation of one single purified antibody half molecule and the result is displayed with the retention time on the horizontal axis and the absorbance on the vertical axis, a homodimer refers to a molecule that is shown in this detection data as a distribution at a higher molecular weight than the distribution presumed to be at the molecular weight of the monomeric antibody half molecule (a distribution at a shorter retention time than the monomer). For specific materials and methods for size exclusion chromatography, those in the Examples are referred to.

In one embodiment, "homodimerization is inhibited" means that, in the above-mentioned detection data of size exclusion chromatography performed using a preparation of a purified antibody half molecule, the vertical height of the peak of a distribution at a higher molecular weight than the distribution presumed to be at the molecular weight of the monomeric antibody half molecule is lower than the height of the distribution presumed to be at the molecular weight of the monomeric antibody half molecule. In a specific embodiment, the height of the distribution at a higher molecular weight than the distribution presumed to be at the molecular weight of the monomeric antibody half molecule is 50% or lower, 40% or lower, 30% or lower, 20% or lower, 10% or lower, 5% or lower, 2% or lower, or 1% or lower than that of the distribution at the estimated molecular weight of the monomeric antibody half molecule. The lower the upper limit of this percentage, the more inhibition of homodimerization is meant.

In one embodiment, an antibody half molecule may be an antibody half molecule of a single domain antibody, or may be an antibody half molecule containing an L chain. The single domain antibody is as defined in "1. Definitions" above. The L chain contained in the antibody half molecule is described later.

### a. Hinge region

In one aspect, at least one cysteine residue in the hinge region is substituted with another amino acid residue. The cysteine residue may be a cysteine residue in the hinge region at any position according to the EU numbering system as long as disulfide bond formation between H chains can be inhibited by the substitution.

In one embodiment, the position(s) of a cysteine residue(s) to be substituted with another amino acid in the hinge region is either or both of position 226 and position 229 according to the EU numbering system. In a specific embodiment, the positions are both position 226 and position 229 according to the EU numbering system.

The other amino acid residue to substitute for a cysteine residue in the hinge region is not specifically limited, and may be any amino acid residue as long as disulfide bond formation between the H chains of two antibody half molecules is inhibited by the substitution. In a specific embodiment, disulfide bonds are not substantially formed between the H chains of two antibody half molecules.

In one embodiment, the other amino acid residue may be any amino acid residue with which a protein *in vivo* normally does not form a disulfide bond *in vivo,* and examples include an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a tryptophan residue, a tyrosine residue, and a valine residue. In a specific embodiment, the other amino acid residue is a serine residue.

In another embodiment, a hinge region may have alterations other than those at positions 226 and 229 according to the EU numbering system. In this case, the amino acid sequence of the hinge region has a homology (sequence identity) of 70% or higher, 75% or higher, 80% or higher, 85% or higher, 90% or higher, or 95% or higher with a corresponding region in a mammalian native antibody. If a hinge region has a homology at the lower limit of these percentages, the hinge region is derived from that mammalian native antibody. In a specific embodiment, such mammals include rodents, rabbits, and primates. Primates include, in particular, humans. In a specific embodiment, such native antibodies include IgG. IgG includes human IgG or mouse IgG.

In a specific embodiment, a hinge region is derived from human IgG. When the hinge region is derived from human IgG, the occurrence of anti-antibodies is more likely to be avoided when the antibody half molecule is used in a pharmaceutical for humans. The subclasses of human IgG include human IgG1, human IgG2, human IgG3, and human IgG4.

### b. CH2 region

In one aspect, the amino acid residue at position 334 according to the EU numbering system in the CH2 region is an amino acid residue other than an arginine residue and a lysine residue. Alternatively, the amino acid at position 334 may be deleted. When substituting the amino acid residue at position 334, a person skilled in the art can select any amino acid residue other than an arginine residue and a lysine residue as a substitute amino acid residue.

In addition to any of the substitutions in the hinge region as described above, and destabilization of the conformational interface in the CH3 region (CH3 interface) as described later, the amino acid residue at position 334 according to the EU numbering system in the CH2 region being any amino acid residue other than an arginine residue and a lysine residue leads to inhibition of the homodimerization of the antibody half molecule. In particular, if the homodimerization of the antibody half molecule is not sufficiently inhibited only by the substitutions in the hinge region and the destabilization of the conformational interface in the CH3 region, alteration of the amino acid residue at position 334 according to the EU numbering system in the CH2 region to an amino acid residue other than an arginine residue and a lysine residue will promote the inhibition of the homodimerization of the antibody half molecule.

Examples of the amino acid residue other than an arginine residue and a lysine residue include a histidine residue, a cysteine residue, a phenylalanine residue, a glycine residue, an isoleucine residue, a methionine residue, an asparagine residue, a proline residue, a glutamine residue, a serine residue, a threonine residue, a tryptophan residue, a tyrosine residue, an alanine residue, a leucine residue, a valine residue, an aspartic acid residue, and a glutamic acid residue. In one embodiment, the amino acid residue is a histidine residue, a cysteine residue, a phenylalanine residue, a glycine residue, an isoleucine residue, a methionine residue, an asparagine residue, a proline residue, a glutamine residue, a serine residue, a threonine residue, a tryptophan residue, and a tyrosine residue.

In a specific embodiment, the amino acid residue is a histidine residue. If the amino acid residue at position 334 according to the EU numbering system in the CH2 region is a histidine residue, administration of the antibody half molecule as a pharmaceutical to the living body is expected to result in extended plasma or blood half-life of the antibody half molecule.

In another embodiment, the CH2 region may also have any alterations as long as the amino acid residue at position 334 according to the EU numbering system is an amino acid residue other than an arginine residue and a lysine residue. In this case, the amino acid sequence of the CH2 region has a homology (sequence identity) of 70% or higher, 75% or higher, 80% or higher, 85% or higher, 90% or higher, or 95% or higher with a corresponding region in a mammalian native antibody. If a CH2 region has a homology at the lower limit of these percentages, the CH2 region is derived from that mammalian native antibody. In a specific embodiment, such mammals include rodents, rabbits, and primates. Primates include, in particular, humans. In a specific embodiment, such native antibodies include IgG. IgG includes human IgG or mouse IgG.

In a specific embodiment, a CH2 region is derived from human IgG. When the CH2 region is derived from human IgG, the occurrence of anti-antibodies is more likely to be avoided when the antibody half molecule is used in a pharmaceutical for humans. The subclasses of human IgG include human IgG1, human IgG2, human IgG3, and human IgG4.

### c. CH3 region

In one aspect, the conformational interface in the CH3 region is destabilized. The "conformational interface in the CH3 region" refers to a region on the outer molecular surface of an antibody half molecule that has formed a higher-order structure. In one embodiment, the conformational interface in the CH3 region is a binding region in the CH3 regions of two antibody half molecules when the two molecules form a dimer.

In one embodiment, alterations that destabilize the conformational interface in the CH3 region are known in WO2012/020096 and such. Specific examples include substitution of an amino acid residue(s) at at least one or more positions selected from the group consisting of positions 347, 349, 350, 351, 352, 354, 356, 357, 360, 362, 364, 366, 368, 370, 390, 392, 393, 394, 395, 396, 397, 398, 399, 400, 405, 406, 407, 408, 409, 411, and 439 according to the EU numbering system.

In a specific embodiment, the conformational interface in the CH3 region is destabilized by substitution of either or both of the amino acid residues at position 397 and position 409 according to the EU numbering system.

In another embodiment, the CH3 region may have any alterations as long as the conformational interface is destabilized. In this case, the amino acid sequence of the CH3 region has a homology (sequence identity) of 70% or higher, 75% or higher, 80% or higher, 85% or higher, 90% or higher, or 95% or higher with a corresponding region in a mammalian native antibody. If a CH3 region has a homology at the lower limit of these percentages, the CH3 region is derived from that mammalian native antibody. In a specific embodiment, such mammals include rodents, rabbits, and primates. Primates include, in particular, humans. In a specific embodiment, such native antibodies include IgG. IgG includes human IgG or mouse IgG.

In a specific embodiment, a CH3 region is derived from human IgG. When the CH3 region is derived from human IgG, the occurrence of anti-antibodies is more likely to be avoided when the antibody half molecule is used in a pharmaceutical for humans. The subclasses of human IgG include human IgG1, human IgG2, human IgG3, and human IgG4.

### d. CH1 region

In one embodiment, an antibody half molecule may or may not further contain a CH1 region.

In another embodiment, a CH1 region may have any alterations. In this case, the amino acid sequence of the CH1 region has a homology (sequence identity) of 70% or higher, 75% or higher, 80% or higher, 85% or higher, 90% or higher, or 95% or higher with a corresponding region in a mammalian native antibody. If a CH1 region has a homology at the lower limit of these percentages, the CH1 region is derived from that mammalian native antibody. In a specific embodiment, such mammals include rodents, rabbits, and primates. Primates include, in particular, humans. In a specific embodiment, such native antibodies include IgG. IgG includes human IgG or mouse IgG.

In a specific embodiment, a CH1 region is derived from human IgG. When the CH1 region is derived from human IgG, the occurrence of anti-antibodies is more likely to be avoided when the antibody half molecule is used in a pharmaceutical for humans. The subclasses of human IgG include human IgG1, human IgG2, human IgG3, and human IgG4.

In the present embodiment, an antibody half molecule may contain a constant region portion. A constant region portion contains an Fc region portion. When an antibody half molecule contains an Fc region portion or a constant region portion, additional alterations may be added to the Fc region portion or constant region portion to improve or reduce the effector functions it originally has. Specifically, such alterations include those that enhance or reduce the binding affinity for FcyR, FcRn, or C1q, but are not limited thereto.

### e. Antigen-binding region of H chain

In one embodiment, an antibody half molecule may or may not further contain an antigen-binding region of H chain. This antigen-binding region binds to a particular epitope. The antigen-binding region may bind to a single epitope or multiple epitopes. When the antigen-binding region binds to multiple epitopes, it may bind to multiple sites within the same antigen, or bind to different antigens, or both.

In another embodiment, the antigen-binding region of H chain may be derived from human or non-human animals. In a specific embodiment, the antigen-binding region is derived from human IgG. The antigen-binding region derived from human IgG includes an antigen-binding region of a human IgG H chain, a chimeric antigen-binding region between a human IgG H chain and a non-human animal-derived IgG (e.g. mouse IgG) H chain, and an antigen-binding region humanized from a non-human animal-derived IgG (e.g. mouse IgG) H chain. When the antigen-binding region is derived from human IgG, the occurrence of anti-antibodies is more likely to be avoided when the antibody half molecule is used in a pharmaceutical for humans.

In one embodiment, the antigen to which an antigen-binding region of H chain specifically binds is arbitrarily selected by a person with ordinary skilled in the art. Such antigens include, for example, proteins expressed in target cells. Such proteins are preferably antigens expressed in aberrant cells causing a target disease. Such antigens expressed in aberrant cells are preferably membrane proteins. An antigen-binding region of H chain preferably specifically binds to the extracellular region of such a membrane protein.

The antigen is not particularly limited and may be any antigen. Examples of the antigen include, for example, a receptor or a fragment thereof, a cancer antigen, an MHC antigen, and a differentiation antigen, but are not particularly limited thereto.

Examples of the receptors include receptors belonging to the hematopoietic factor receptor family, cytokine receptor family, tyrosine kinase-type receptor family, serine/threonine kinase-type receptor family, TNF receptor family, G protein-coupled receptor family, GPI-anchored receptor family, tyrosine phosphatase-type receptor family, adhesion factor family, hormone receptor family, and such. Reports on the receptors belonging to these receptor families and their characteristics can be found in various sources of documents, for example, in Cooke BA., King RJB., van der Molen HJ. ed. New Comprehensive Biochemistry Vol.18B "Hormones and their Actions Part II" pp.1-46 (1988) Elsevier Science Publishers BV, New York, USA; Patthy L. (1990) Cell, 61: 13-14; Ullrich A., et al. (1990) Cell, 61: 203-212; Massagul J. (1992) Cell, 69: 1067-1070; Miyajima A., et al. (1992) Annu. Rev. Immunol., 10: 295-331; Taga T. and Kishimoto T. (1992) FASEB J., 7: 3387-3396; Fantl WI., et al. (1993) Annu. Rev. Biochem., 62: 453-481; Smith CA., et al. (1994) Cell, 76: 959-962; Flower DR. (1999) Biochim. Biophys. Acta, 1422: 207-234; Miyasaka M. ed. Cell Technology, Handbook Series "Handbook for adhesion factors" (1994) Shujunsha, Tokyo, Japan; and such. Examples of specific receptors belonging to the above-mentioned receptor families include human or mouse erythropoietin (EPO) receptor, human or mouse granulocyte-colony stimulating factor (G-CSF) receptor, human or mouse thrombopoietin (TPO) receptor, human or mouse insulin receptor, human or mouse Flt-3 ligand receptor, human or mouse platelet-derived growth factor (PDGF) receptor, human or mouse interferon (IFN)-α or -β receptor, human or mouse leptin receptor, human or mouse growth hormone (GH) receptor, human or mouse interleukin (IL)-10 receptor, human or mouse insulin-like growth factor (IGF)-I receptor, human or mouse leukemia inhibitory factor (LIF) receptor, and human or mouse ciliary neurotrophic factor (CNTF) receptor (hEPOR: Simon, S. et al. (1990) Blood 76, 31-35; mEPOR: D'Andrea, AD. et al. (1989) Cell 57, 277-285; hG-CSFR: Fukunaga, R. et al. (1990) Proc. Natl. Acad. Sci. USA. 87, 8702-8706; mG-CSFR: Fukunaga, R. et al. (1990) Cell 61, 341-350; hTPOR: Vigon, I. *et al.* (1992) 89, 5640-5644.; mTPOR: Skoda, RC. *et al.* (1993) 12, 2645-2653; hlnsR: Ullrich, A. et al. (1985) Nature 313, 756-761; hFlt-3: Small, D. et al. (1994) Proc. Natl. Acad. Sci. USA. 91, 459-463; hPDGFR: Gronwald, RGK. et al. (1988) Proc. Natl. Acad. Sci. USA. 85, 3435-3439; hIFN α/β R: Uze, G. et al. (1990) Cell 60, 225-234; and Novick, D. et al. (1994) Cell 77, 391-400).

Cancer antigens are antigens that are expressed as cells become malignant, and are also called tumor-specific antigens. Furthermore, abnormal sugar chains that appear on cell surfaces and protein molecules when the cells become cancerous are also cancer antigens and are specifically called as carcinoma associated carbohydrate antigen. Examples of cancer antigens include CA19-9, CA15-3, and sialyl SSEA-1 (SLX).

MHC antigens can be classified broadly into MHC class I antigens and MHC class II antigens: MHC class I antigens include HLA-A, -B, -C, -E, -F, -G, and -H; and MHC class II antigens include HLA-DR, -DQ, and -DP.

Differentiation antigens include CD1, CD2, CD3, CD4, CD5, CD6, CD7, CD8, CD10, CD11a, CD11b, CD11c, CD13, CD14, CD15s, CD16, CD18, CD19, CD20, CD21, CD23, CD25, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD71, CD73, CD95, CD102, CD106, CD122, CD126, and CDw130.

### f. L chain

In one embodiment, an antibody half molecule may or may not contain an L chain. The L chain may be a whole L chain or a fragment thereof. For example, it may contain either or both of an antigen-binding region portion and a constant region portion of L chain.

When an antibody half molecule contains an antigen-binding region of L chain, this antigen-binding region binds to a particular epitope. The antigen-binding region may bind to a single epitope or multiple epitopes. When the antigen-binding region binds to multiple epitopes, it may bind to multiple sites within the same antigen, or bind to different antigens, or both.

An antigen-binding region of L chain may be derived from human or non-human animals. In a specific embodiment, the antigen-binding region is derived from human IgG. The antigen-binding region derived from human IgG includes an antigen-binding region of a human IgG L chain, a chimeric antigen-binding region between a human IgG L chain and a non-human animal-derived IgG (e.g. mouse IgG) L chain, and an antigen-binding region humanized from a non-human animal-derived IgG (e.g. mouse IgG) L chain. When the antigen-binding region is derived from human IgG, the occurrence of anti-antibodies is more likely to be avoided when the antibody half molecule is used in a pharmaceutical for humans.

In one embodiment, the antigen that an antigen-binding region of L chain specifically binds is arbitrarily selected by a person with ordinary skilled in the art. Such antigens include, for example, proteins expressed in target cells. Such proteins are preferably antigens expressed in aberrant cells causing a target disease. Such antigens expressed in aberrant cells are preferably membrane proteins. An antigen-binding region of L chain preferably specifically binds to the extracellular region of such a membrane protein.

The antigen is not particularly limited and may be any antigen. Examples of the antigen include those as mentioned above for the antigen-binding region of H chain. In a specific embodiment, when an antibody half molecule contains an H chain antigen-binding region and an L chain antigen-binding region, the antigens to which the two antigen-binding regions bind may or may not be identical.

When an antibody half molecule contains a constant region portion of L chain, the constant region may have any alterations. In this case, the amino acid sequence of the constant region has a homology (sequence identity) of 70% or higher, 75% or higher, 80% or higher, 85% or higher, 90% or higher, or 95% or higher with a corresponding region in a mammalian native antibody. If a constant region has a homology at the lower limit of these percentages, the constant region is derived from that mammalian native antibody. In a specific embodiment, such mammals include rodents, rabbits, and primates. Primates include, in particular, humans. In a specific embodiment, such native antibodies include IgG. IgG includes human IgG or mouse IgG.

In a specific embodiment, the constant region is derived from human IgG. When the constant region is derived from human IgG, the occurrence of anti-antibodies is more likely to be avoided when the antibody half molecule is used in a pharmaceutical for humans. The subclasses of human IgG include human IgG1, human IgG2, human IgG3, and human IgG4.

### g. Specific examples of antibody half molecule

In one embodiment, an antibody half molecule may contain each one of the above-mentioned hinge region, CH2 region, and CH3 region, and optionally CH1 region, H chain antigen-binding region, and L chain, may contain two or more of any of these, or may contain two or more of all of these, as long as its homodimerization is inhibited.

In the antibody half molecule, the above-mentioned hinge region, CH2 region, CH3 region, and optionally CH1 region, H chain antigen-binding region, and L chain, may be present within a single polypeptide or separately present in multiple polypeptides. When these are separately present in multiple polypeptides, those polypeptides are linked with each other by a chemical bond such as a covalent bond.

In one embodiment, at least one selected from the group consisting of the CH1 region, hinge region, CH2 region, and CH3 region is derived from human IgG. In a specific embodiment, the CH1 region, hinge region, CH2 region, and CH3 region are derived from human IgG.

In another embodiment, the L chain is derived from human IgG.

In yet another embodiment, the CH1 region, hinge region, CH2 region, CH3 region, and L chain are derived from human IgG.

### h. Uses

An antibody half molecule can be used for known uses. For example, such uses include those described in WO2012/020096. Specific uses of an antibody half molecule include treatment and diagnosis.

In one embodiment, when an antibody half molecule is used for treatment, for example, the antibody half molecule is used in such a way that its effector functions are partially or completely inhibited when it acts in the monomeric form, but it exerts stronger effector functions against aberrant cells that highly express the target antigen of the antibody half molecule, than when it acts in the monomeric form. This use utilizes, for example, a mechanism by which while the antibody half molecule hardly or does not exert effector functions when bound to the cell surface in the monomeric form, it exerts effector functions when bound to the target antigen on the cell surface at a certain density or higher, where the antibody half molecule forms a dimer using the antigen as a scaffold.

More specifically, two antibody half molecules that target different antigens are used in combination. By targeting different antigens, the antibody half molecules have improved target specificity for aberrant cells. In this case, these antibody half molecules are preferably not linked via a covalent bond and form a heterodimer more readily than a homodimer when mixed in liquid. In addition, it is preferred that aberrant cells express both of the target antigens of these antibody half molecules but normal cells do not express either one of them. It is more preferred that aberrant cells express both antigens but normal cells express neither of them.

Hereinbelow, specific embodiments of the uses of antibody half molecules are described in detail, referring to the two antibody half molecules as "first antibody half molecule" and "second antibody half molecule", respectively, and referring to the antigen to which the first antibody half molecule specifically binds as "first antigen", and the antigen to which the second antibody half molecule specifically binds as "second antigen".

The first antibody half molecule and the second antibody half molecule are not linked by a covalent bond. When the two antibody half molecules are present in the same composition, the first antibody half molecule and the second antibody half molecule may interact with each other as long as they are not covalently linked. Examples of such non-covalent interactions include hydrogen bond and intermolecular bond. The amount of such interaction is preferably small. The smaller the amount, the more the side effects are reduced.

In one embodiment, the molar ratio of the amount of binding between the first antibody half molecule and the second antibody half molecule measured by surface plasmon resonance can be used as an indicator of the interaction. For example, when the affinity between the two antibody half molecules is measured in surface plasmon resonance using a sensor chip on which 50 pg of the first antibody half molecule is immobilized per 1 mm² and a measurement solution containing 2.5 mg/mL of the second antibody half molecule, the binding amount of the second antibody half molecule to the first antibody half molecule is within the range of 1:0.1 to 1:0.9 in terms of molar ratio.

As the upper limit value of the binding amount of the second antibody half molecule, the molar ratio may be 1:0.9 or less, preferably 1:0.8 or less, more preferably 1:0.7 or less, still more preferably 1:0.65 or less, most preferably 1:0.5 or less. The lower the upper limit value, the less heterodimer is formed under conditions in which cells expressing the first antigen and the second antigen are not present, and side effects are further reduced.

On the other hand, as the lower limit value of the binding amount of the second antibody half molecule, the molar ratio may be 1:0.1 or more, preferably 1:0.14 or more, more preferably 1:0.17 or more, still more preferably 1: 0.2 or more, and most preferably 1: 0.23 or more. The higher the lower limit value, the more easily heterodimers of the first antibody half molecule and the second antibody half molecule are formed on the surface of cells expressing the first antigen and the second antigen, and the effector function becomes higher.

Examples of a surface plasmon resonance apparatus include Biacore (registered trademark) T200 (GE Healthcare).

Examples of a measurement solution used for surface plasmon resonance measurement include HBS-EP+10X (GE Healthcare). Since HBS-EP+10X is a measurement solution concentrated 10 times, it is diluted to 1/10th when used. The specific composition of the measurement solution at the time of use is 0.01 M HEPES, 0.15 M NaCl, 3 mM EDTA, 0.05% (v/v) Surfactant P20, pH 7.4. The preferred temperature of the measurement solution at the time of measurement is 25°C.

In one embodiment, the first antibody half molecule and the second antibody half molecule are more likely to form heterodimers than homodimers when mixed in solution. The "homodimer" in this embodiment means a dimer formed by a noncovalent interaction between a first antibody half molecule and a first antibody half molecule or a dimer formed by a noncovalent interaction between a second antibody half molecule and a second antibody half molecule. Moreover, the "heterodimer" is a dimer formed by a noncovalent interaction between a first antibody half molecule and a second antibody half molecule. Examples of the interactions include hydrogen bond and intermolecular bond.

From the viewpoint of reducing side effects, the first antibody half molecule and the second antibody half molecule are preferably less likely to interact in solution. However, since the interaction between the first antibody half molecule and the second antibody half molecule is in a state of equilibrium, interaction may occur when the concentration of the first antibody half molecule and the second antibody half molecule in solution is increased more than the concentration in the pharmaceutical composition suitable for administration to a subject. In that case, the higher the concentration of the first antibody half molecule and the second antibody half molecule, the greater the amount of the interacting first antibody half molecule and second antibody half molecule.

A specific embodiment of the first antibody half molecule and the second antibody half molecule which are more likely to form heterodimers than homodimers when mixed in solution includes an embodiment wherein the first antibody half molecule comprises a first CH3 region and the second antibody half molecule comprises a second CH3 region, and wherein the first CH3 region and the second CH3 region are altered such that they form the heterodimer more easily than the homodimers when mixed in solution. Specific examples of such alterations include at least one of the alterations of (i) to (iii) below:
(i) an alteration where either one of the first CH3 region and the second CH3 region has a positively-charged region and the other has a negatively-charged region, and when the heterodimer is formed, the positively-charged region interacts with the negatively-charged region,
(ii) an alteration where either one of the first CH3 region and the second CH3 region has a convex portion and the other has a concave portion, and when the heterodimer is formed, the convex portion fits into and interacts with the concave portion, and
(iii) an alteration where the first CH3 region and the second CH3 region are altered IgG CH3 region, a part of which is replaced with a part of IgA CH3 region, and when said heterodimer is formed, the replaced part of IgA CH3 region in said first CH3 region interacts with the replaced part of IgA CH3 region in said second CH3 region.

Examples of the alteration of (i) include those disclosed in WO 2006/106905, WO 2009/089004, WO 2010/129304, and WO 2014/084607. An example of a specific method is: altering at least one combination from among the combinations of positions 356 and 439, positions 357 and 370, and position 399 and 409 of the EU numbering system in the amino acid sequence of the heavy chain constant region of the polypeptide having the first antigen-binding activity, to amino acids having the same charge; and altering at least one combination from among the combinations of positions 356 and 439, positions 357 and 370, and position 399 and 409 of the EU numbering system in the heavy chain constant region of the polypeptide having the second antigen-binding activity or not having antigen-binding activity, to amino acids having a charge opposite to that of the polypeptide having the first antigen-binding activity. More specifically, for example, in the amino acid sequences of the heavy chain constant regions of the polypeptide having the first antigen-binding activity and the polypeptide having the second antigen-binding activity, either one of the polypeptides is introduced with a mutation that substitutes Glu at position 356 according to the EU numbering system with Lys, and the other polypeptide is introduced with a mutation that substitutes Lys at position 439 according to the EU numbering system with Glu.

Examples of the alteration of (ii) include those disclosed in WO 96/027011 and Margaret Merchant et al., Nature Biotechnology 1998, 16, 677-681. Examples of specific methods are: the combination of introducing T366Y to one CH3 region and Y407A to the other CH3 region; or the combination of introducing T366W to one CH3 region and Y407A to the other CH3 region, or the combination of introducing F405A to one CH3 region and T394W to the other CH3 region, or the combination of introducing Y407T to one CH3 region and T366Y to the other CH3 region, or the combination of introducing T366Y/F405A to one CH3 region and T394W/Y407T to the other CH3 region, or the combination of introducing T366W/F405W to one CH3 region and T394S/Y407A to the other CH3 region, or the combination of introducing F405W/Y407A to one CH3 region and T366W/T394S to the other CH3 region, or the combination of introducing F405W to one CH3 region and T394S to the other CH3 region, or the combination of introducing T366W to one CH3 region and T366S/L368A/Y407V to the other CH3 region. The alteration of (ii) can be combined with the alteration of (i). Examples of such combinations include those disclosed in WO 2012/058768.

The alteration of (iii) is a technique of using strand-exchange engineered domain CH3s, in which a part of one H chain CH3 region of an antibody is altered to a sequence derived from IgA corresponding to that part, and the complementary part of the other H chain CH3 region is introduced with an IgA-derived sequence corresponding to that part, to efficiently induce the interaction of polypeptides having different sequences by complementary interaction of the CH3 regions (Protein Engineering Design & Selection, 23 ; 195-202, 2010). This known technique can also be used to make it easier to form a heterodimer efficiently. Examples of the alteration of (iii) include the alteration technique disclosed in WO 2007/110205.

As another specific embodiment of the first antibody half molecule and the second antibody half molecule which are more likely to form heterodimers than homodimers when mixed in solution, alterations added to the hinge region portion may be applied. Such alterations include, for example, the alteration technique disclosed in WO 2011/143545.

Either or both of the first CH3 region and the second CH3 region preferably has a substitution of at least one of the amino acid residues at positions 357, 397, and 409 according to the EU numbering system to another amino acid residue. By adding such an alteration, it becomes possible to set the binding amount of the second antibody half molecule to the first antibody half molecule easily within the range of the above-mentioned molar ratio when affinity between the two antibody half molecules is measured using a sensor chip on which 50 pg of the first antibody half molecule is immobilized per 1 mm² and a measurement solution containing 2.5 mg/mL of the second antibody half molecule in the surface plasmon resonance described above.

In one embodiment, when the heterodimer is formed on the surface of cells expressing the first antigen and the second antigen, the binding activity of the heterodimer to FcγR is higher than the binding activity of the monomer of the first antibody half molecule or the monomer of the second antibody half molecule to FcγR, or, when the homodimers are formed, than the binding activity of the homodimers to FcγR. This means, for example, that when the pharmaceutical composition is administered to a subject having cells expressing the first antigen and the second antigen, the heterodimer formed by the first antibody half molecule and the second antibody half molecule that have reached the cell surface leads to greater activation of FcγR than the monomers simply bound to the cell surface or the homodimers formed on the cell surface, and exerts an effector function. This further reduces side effects.

In this embodiment, the FcγR includes, for example, rodent and primate FcγRs and may be any one of these FcγRs. In this embodiment, the FcγR is preferably a rodent and a primate FcγR. Rodents are preferably mice and rats. Primates are preferably cynomolgus monkeys and humans. In this embodiment, the FcγR includes human FcγRs, and rodent and nonhuman primate homologs that are structurally homologous and functionally similar to human FcγRs.

Subclasses of FcγR in this embodiment include human FcγRI, human FcγRII, and human FcγRIII, and rodent and non-human primate homologs thereof. Among these, FcγR is preferably a human FcγRII or human FcγRIII, or a rodent and nonhuman primate homolog thereof, more preferably, human FcγRIII or a rodent and nonhuman primate homolog thereof. The human FcγR is preferably human FcγRII or human FcγRIII, more preferably human FcγRIII.

Human FcγRII in this embodiment is further divided into human FcγRIIA, human FcγRIIB, and human FcγRIIC. Among these, human FcγRII is preferably human FcγRIIB. Human FcγRIII is further divided into human FcγRIIIA and human FcγRIIIB. Among these, human FcγRIII is preferably human FcγRIIIA.

In one embodiment, the effector function under conditions in which cells expressing the first antigen and the second antigen are present is higher than that under conditions in which cells expressing the first antigen without expressing the second antigen or cells expressing the second antigen without expressing the first antigen are present. This further reduces side effects.

The effector function is preferably ADCC and CDC, more preferably ADCC.

### 3. Methods for producing antibody half molecules

In one aspect, the present invention provides methods for producing antibody half molecules.

Antibody half molecules are produced by a general method for obtaining a protein. Antibody half molecules are generally obtained by expressing them in host cells using nucleic acids encoding them. The antibody half molecule expressed in the host cell is usually recovered from the host cell and purified. A specific production method is described below.

The nucleic acids are ordinarily carried by (inserted into) suitable vectors and then introduced into host cells. These vectors are not particularly limited so long as the inserted nucleic acid is stably maintained. For example, when using *E. coli* as the host, the cloning vector is preferably a pBluescript vector (Stratagene) and such, but various commercially available vectors may be used. Expression vectors are particularly useful as vectors for producing the antibody half molecules. Expression vectors are not particularly limited so long as they can express polypeptides in test tubes, *E. coli,* cultured cells, or individual organisms. For example, preferred vectors include pBEST vector (Promega) for expression in test tubes, pET vector (Invitrogen) for *E*. *coli*, pME18S-FL3 vector (GenBank Accession No. AB009864) for cultured cells, and pME18S vector (Mol. Cell Biol. 8:466-472(1998)) for individual organisms. Insertion of a DNA of the present invention into vectors can be performed by standard methods such as ligase reactions using restriction enzyme sites (Current protocols in Molecular Biology edit. Ausubel et al. (1987) Publish. John Wiley & Sons. Section 11.4-11.11).

The above-mentioned host cells are not particularly limited, and various host cells can be used, depending on the purpose. Cells used for expressing the antibody half molecules include bacterial cells (for example, *Streptococcus, Staphylococcus, E. coli, Streptomyces,* and *Bacillus subtilis*), fungal cells (for example, yeast and *Aspergillus*), insect cells (for example, *Drosophila* S2 and *Spodoptera* SF9), animal cells (for example, CHO, COS, HeLa, C127, 3T3, BHK, HEK293, Bowes melanoma cell), and plant cells. Vectors can be introduced into host cells using known methods, such as the calcium phosphate precipitation method, electroporation method (Current protocols in Molecular Biology edit. Ausubel et al. (1987) Publish. John Wiley & Sons. Section 9.1-9.9), lipofectamine method (GIBCOBRL), and microinjection method.

For secreting host cell-expressed antibody half molecules into the lumen of the endoplasmic reticulum, periplasmic space, or extracellular environment, suitable secretion signals can be incorporated into the antibody half molecules of interest. These signals may be intrinsic or foreign to the antibody half molecules of interest.

When the antibody half molecules of the present invention are secreted into the culture media, the antibody half molecules produced by the above-mentioned method can be harvested by collecting the media. When the antibody half molecules of the present invention are produced inside cells, first, the cells are lysed, and then these antibody half molecules are collected.

The antibody half molecules of the present invention can be collected and purified from recombinant cell cultures by using known methods, including ammonium sulfate or ethanol precipitation, acidic extraction, anion or cation exchange chromatography, phosphocellulose chromatography, hydrophobic interaction chromatography, affinity chromatography, hydroxyapatite chromatography, and lectin chromatography.

### 4. Pharmaceutical compositions

In one aspect, the present invention provides pharmaceutical compositions comprising the above-mentioned antibody half molecules.

### a. Other components

The pharmaceutical compositions may contain other components besides the antibody half molecules. Examples of other components include pharmaceutically acceptable carriers.

The pharmaceutical compositions can be formulated by methods known to those skilled in the art. For example, such pharmaceutical compositions can be used parenterally, as injections which are sterile solutions or suspensions including an antibody along with water or another pharmaceutically acceptable liquid. For example, such compositions may be formulated as unit doses that meet the requirements for the preparation of pharmaceuticals by appropriately combining the antibody with pharmaceutically acceptable carriers or media, specifically with sterile water, physiological saline, a vegetable oil, emulsifier, suspension, detergent, stabilizer, flavoring agent, excipient, vehicle, preservative, binder, or such. In such preparations, the amount of active ingredient is adjusted such that the dose falls within an appropriately pre-determined range.

Sterile compositions for injection can be formulated using vehicles such as distilled water for injection, according to standard protocols for formulation.

Aqueous solutions for injection include, for example, physiological saline and isotonic solutions containing dextrose or other adjuvants (for example, D-sorbitol, D-mannose, D-mannitol, and sodium chloride). Appropriate solubilizers, for example, alcohols (ethanol and such), polyalcohols (propylene glycol, polyethylene glycol, and such), non-ionic detergents (polysorbate 80, HCO-50, and such), may be used in combination.

Oils include sesame and soybean oils. Benzyl benzoate and/or benzyl alcohol can be used in combination as solubilizers. Buffers (for example, phosphate buffer and sodium acetate buffer), soothing agents (for example, procaine hydrochloride), stabilizers (for example, benzyl alcohol and phenol), and/or antioxidants can also be combined. Prepared injectables are generally filled into appropriate ampules.

### 5. Dosage form

The pharmaceutical compositions are preferably administered parenterally. For example, the compositions may be injections, transnasal compositions, transpulmonary compositions or transdermal compositions. For example, such compositions can be administered systemically or locally by intravenous injection, intramuscular injection, intraperitoneal injection, subcutaneous injection, or such.

### c. Target diseases

Target diseases for pharmaceutical compositions are not particularly limited, and preferably are diseases caused by pathogenic cells expressing both antigens targeted by two antibody half molecules. In other words, such diseases are those in which two antibody half molecules preferably form a heterodimer on the cell surface and exert effector functions.

Specific target diseases include, for example, cell proliferative diseases, immune-enhanced diseases, and infectious diseases. Cell proliferative diseases include tumors. Immune-enhanced diseases include autoimmune diseases. Infectious diseases include bacterial infections and viral infections.

A pharmaceutical composition is administered for a target disease by a person skilled in the art using any method. When multiple antibody half molecules are used for treatment, they may be contained in one formulation and administered, or contained in separate formulations and administered at the same time or different times.

### 5. Methods for inhibiting homodimerization of antibody half molecules

In one aspect, the present invention provides a method for inhibiting homodimerization of an antibody half molecule comprising a hinge region, CH2 region and CH3 region in the production of the antibody half molecule, which comprises (a), (b) and (c) below:
(a) substitution of at least one cysteine residue in the hinge region with another amino acid;
(b) substitution of the amino acid residue at position 334 according to the EU numbering system in the CH2 region with an amino acid residue other than an arginine residue and a lysine residue; and
(c) destabilization of a conformational interface in the CH3 region.

In this method, the details of the position of the cysteine residue, the other amino acids, and such in (a), the amino acid residues other than arginine and lysine residues in (b), and the destabilization of the conformational interface in (c), are as described in "2. Antibody half molecules" above.

### [Examples]

### [Example 1] Production of expression vectors for antibody half molecules and expression and purification of antibody half molecules

Expression vectors were constructed, in which amino acid substitutions were introduced by methods known to those skilled in the art using QuikChange Site-Directed Mutagenesis Kit (Stratagene), PCR, In fusion Advantage PCR cloning kit (TAKARA), or such. The nucleotide sequences of the resulting expression vectors were determined by methods known to those skilled in the art. The produced plasmids were transiently introduced into Expi293 cells (Invitrogen) to express antibody half molecules. Antibody half molecules were purified from the obtained culture supernatants by methods known to skilled in the art using MonoSpin® ProA 96-well plate type (GL Sciences). The concentration of purified antibody half molecules was determined by measuring the absorbance at 280 nm using a spectrophotometer and calculating the antibody half molecule concentration from the obtained value using an extinction coefficient calculated by the PACE method (Protein Science 1995; 4: 2411-2423).

### [Example 2] Production of antibodies the dimerization of which is destabilized

In order to test whether half molecule antibodies can be obtained by destabilizing the regions involved in antibody dimerization, alterations for destabilizing the hinge region and the CH3 interface were introduced. The abbreviation and name of the produced antibody variant, the introduced alterations, and the SEQ ID NOs of the antibody variant are shown in Table 1.

**[Table 1]**

| Abbreviation | Name | Alteration | Heavy chain SEQ ID NO: | Light chain SEQ ID NO: |
|---|---|---|---|---|
| K334K | MRAH-G1d.dh3.dCH3 | C226S/C229S/V397Y/K409D | 9 | 8 |

### [Example 3] Evaluation of the molecular weight of antibodies the dimerization of which is destabilized

The molecular weight of antibodies altered to destabilize dimerization were evaluated by size exclusion chromatography. Analysis was performed by methods known to those skilled in the art, using ACQUITY® UPLC H-Class (Waters), which is an HPLC, for measurement and using SuperSW3000 (TOSOH) as the column. The concentration of antibody half molecule protein was 0.1 mg/mL, and 10 µL was injected.

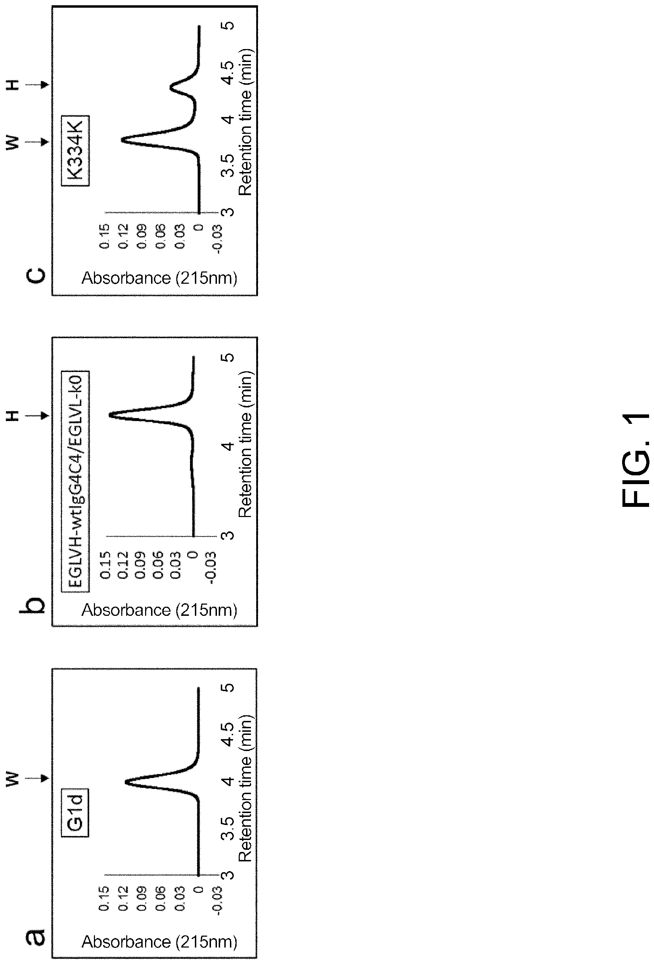

For the whole antibody control (peak W in Fig. 1a), the anti-human interleukin 6 receptor antibody disclosed in WO 2009/125825 was used. MRAH (SEQ ID NO: 1) was used for the H chain variable region, and a native IgG1 sequence (SEQ ID NO: 2) was used for the H chain constant region. MRAL (SEQ ID NO: 3) was used for the L chain variable region, and a native κ chain sequence, k0 (SEQ ID NO: 4), was used for the L chain constant region. For the antibody half molecule state control (peak H in Fig. 1b), EGLVH (SEQ ID NO: 5), which is the H chain variable region portion of the anti-human epiregulin antibody described in WO 2013/100120, was used for the H chain variable region, and wtIgG4C4 (SEQ ID NO: 6), which has the alterations described in a report by Lu *et al.* (Shan L, Colazet M, Rosenthal KL, Yu XQ, Bee JS, Ferguson A, Damschroder MM, Wu H, Dall'Acqua WF, Tsui P, Oganesyan V. (2016) Generation and Characterization of an IgG4 Monomeric Fc Platform. PLoS One. 2016 Aug 1; 11(8)), was used for the H chain constant region portion. EGLVL (SEQ ID NO: 7), which is the L chain variable region portion of the anti-human epiregulin antibody described in WO 2013/100120, was used for the L chain variable region, and a native κ chain sequence, k0 (SEQ ID NO: 4), was used for the L chain constant region. For antibody half molecules other than the above-mentioned controls, MRAH (SEQ ID NO: 1) was used for the H chain variable region, and MRAL-k0 (SEQ ID NO: 8) was used for the L chain for all molecules, and measurement was carried out.

As a result of the measurement, as shown in Fig. 1, the variant with destabilized hinge region and CH3 interface showed a mixture of the antibody half molecule (peak H) and the whole antibody (peak W) (Fig. 1c).

### [Example 4] Production of the molecular weight of position 334 variants

In order to test the involvement of position 334 (EU numbering) in the formation of antibody half molecules, alterations to substitute position 334 with other amino acid residues were introduced into the dimerization-destabilized antibody produced in Example 2. The abbreviations and names of the produced antibody variants, the introduced alterations at position 334, and the SEQ ID NOs of the antibody variants, are shown in Table 2.

**[Table 2]**

| Abbreviation | Name | Alteration at position 334 | Heavy chain SEQ ID NO: | Light chain SEQ ID NO: |
|---|---|---|---|---|
| K334A | G1d.dh3.dCH3.K334A | A | 10 | 8 |
| K334C | G1d.dh3.dCH3.K334C | C | 11 | 8 |
| K334D | G1d.dh3.dCH3.K334D | D | 12 | 8 |
| K334E | G1d.dh3.dCH3.K334E | E | 13 | 8 |
| K334F | G1d.dh3.dCH3.K334F | F | 14 | 8 |
| K334G | G1d.dh3.dCH3.K334G | G | 15 | 8 |
| K334H | G1d.dh3.dCH3.K334H | H | 16 | 8 |
| K334I | G1d.dh3.dCH3.K334I | I | 17 | 8 |
| K334L | G1d.dh3.dCH3.K334L | L | 18 | 8 |
| K334M | G1d.dh3.dCH3.K334M | M | 19 | 8 |
| K334N | G1d.dh3.dCH3.K334N | N | 20 | 8 |
| K334P | G1d.dh3.dCH3.K334P | P | 21 | 8 |
| K334Q | G1d.dh3.dCH3.K334Q | Q | 22 | 8 |
| K334R | G1d.dh3.dCH3.K334R | R | 23 | 8 |
| K334S | G1d.dh3.dCH3.K334S | S | 24 | 8 |
| K334T | G1d.dh3.dCH3.K334T | T | 25 | 8 |
| K334V | G1d.dh3.dCH3.K334V | V | 26 | 8 |
| K334W | G1d.dh3.dCH3.K334W | W | 27 | 8 |
| K334Y | G1d.dh3.dCH3.K334Y | Y | 28 | 8 |

### [Example 5] Evaluation of the molecular weight of position 334 variants

The molecular weight of antibodies altered to destabilize dimerization were evaluated by size exclusion chromatography. Analysis was performed by methods known to those skilled in the art, using ACQUITY® UPLC H-Class (Waters), which is an HPLC, for measurement and using SuperSW3000 (TOSOH) as the column. The concentration of antibody half molecule protein was 0.1 mg/mL, and 10 µL was injected.

As a result of the measurement, as shown in Fig. 2, a mixture of the antibody half molecule (peak H) and the whole antibody (peak W) was seen in the case of substituting position 334 with R (Fig. 2n), and little or no whole antibody was seen in the other cases.

## Claims

1. An antibody half molecule comprising a hinge region, a CH2 region, and a CH3 region, wherein at least one cysteine residue in the hinge region is substituted with another amino acid residue, wherein the amino acid residue at position 334 according to the EU numbering system in the CH2 region is an amino acid residue other than an arginine residue and a lysine residue, and wherein a conformational interface in the CH3 region is destabilized.

2. The antibody half molecule of claim 1, wherein homodimerization of the antibody half molecule is inhibited.

3. The antibody half molecule of claim 1 or 2, wherein the amino acid residue at position 334 according to the EU numbering system is selected from the group consisting of a histidine residue, a cysteine residue, a phenylalanine residue, a glycine residue, an isoleucine residue, a methionine residue, an asparagine residue, a proline residue, a glutamine residue, a serine residue, a threonine residue, a tryptophan residue, a tyrosine residue, an alanine residue, a leucine residue, a valine residue, an aspartic acid residue, and a glutamic acid residue.

4. The antibody half molecule of any one of claims 1 to 3, wherein the at least one cysteine residue is either or both of position 226 and position 229 according to the EU numbering system.

5. The antibody half molecule of any one of claim 1 to 4, wherein the conformational interface in the CH3 region is destabilized by substitution of either or both of amino acid residues at position 397 and position 409 according to the EU numbering system.

6. The antibody half molecule of any one of claims 1 to 5, which further comprises an antigen-binding region of H chain.

7. The antibody half molecule of any one of claims 1 to 6, which further comprises a CH1 region.

8. The antibody half molecule of claim 7, wherein at least one selected from the group consisting of the CH1 region, the hinge region, the CH2 region, and the CH3 region is derived from human IgG.

9. The antibody half molecule of any one of claims 1 to 8, which is an antibody half molecule of a single domain antibody.

10. The antibody half molecule of any one of claims 1 to 8, which further comprises an antibody L chain.

11. The antibody half molecule of claim 10, wherein the L chain is derived from human IgG.
